(19) Europäisches Patentamt
European Patent Office
Office européen des brevets

(11) Numéro de publication : **0 025 383**
**B1**

(12) **FASCICULE DE BREVET EUROPÉEN**

(45) Date de publication du fascicule du brevet : **22.09.82**

(51) Int. Cl.[3] : **C 07 C 69/74**, C 07 C 67/343// C07D231/06, C07D231/14

(21) Numéro de dépôt : **80401231.8**

(22) Date de dépôt : **28.08.80**

(54) **Nouveaux dérivés substitués du cyclopropène, leur préparation et leur application dans la synthèse de précurseurs de l'acide di cis chrysanthémique.**

(30) Priorité : **10.09.79 FR 7922559**

(43) Date de publication de la demande : **18.03.81 (Bulletin 81/11)**

(45) Mention de la délivrance du brevet : **22.09.82 Bulletin 82/38**

(84) Etats contractants désignés : **CH DE GB LI NL**

(56) Documents cités : **Néant**

(73) Titulaire : **ROUSSEL-UCLAF**
**35, boulevard des Invalides**
**F-75007 Paris (FR)**

(72) Inventeur : **Franck-Neumann, Michel**
**4, rue Andrieux**
**F-67000 Strasbourg (FR)**
Inventeur : **Miesch, Michel**
**42a, rue des Grains**
**F-68200 Mulhouse (FR)**

(74) Mandataire : **Burlot, Pierre et al**
**Boite postale no 9 102, route de Noisy**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

EP 0 025 383 B1

Nouveaux dérivés substitués du cyclopropène, leur préparation et leur application dans la synthèse de précurseurs de l'acide dl cis chrysanthémique

La présente invention concerne de nouveaux dérivés substitués du cyclopropène, leur préparation et leur application dans la synthèse de précurseur de l'acide dl cis chrysanthémique.

L'invention a pour objet les composés de formule I

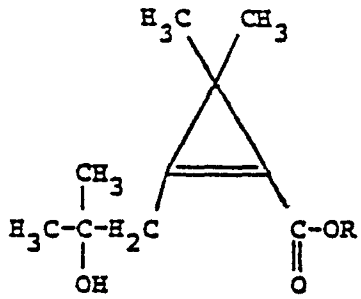

(I)

dans laquelle R représente un radical alcoyle renfermant de I à 6 atomes de carbone.

Dans la formule I, R peut représenter un radical méthyle, éthyle, propyle linéaire ou ramifié, butyle linéaire ou ramifié, pentyle linéaire ou ramifié ou hexyle linéaire ou ramifié.

Parmi les composés de formule I, on peut citer notamment le 3,3-diméthyl-2-(2-hydroxy-2-méthylpropyl)-I-cyclopropène-I-carboxylate d'éthyle.

L'invention a également pour objet un procédé de préparation des composés de formule I, tels que définis précédemment, caractérisé en ce que l'on fait réagir un composé de formule II

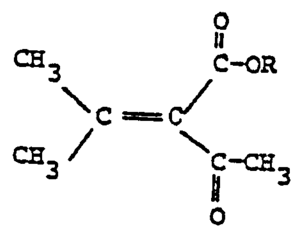

(II)

dans laquelle R est défini comme précédemment, avec une base forte puis avec un organolithien, fait réagir le composé obtenu avec de l'acétone pour obtenir le composé de formule III

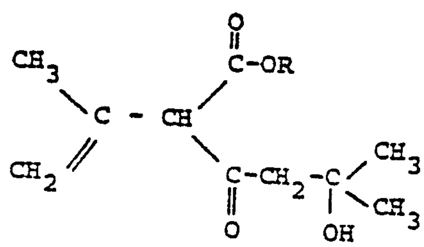

(III)

isomérise le composé de formule III par un agent basique ou par un agent acide pour obtenir le composé de formule IV

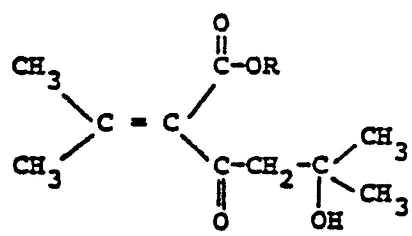

(IV)

fait réagir le composé de formule IV avec de l'hydrazine dans l'acide acétique, pour obtenir le composé de formule V

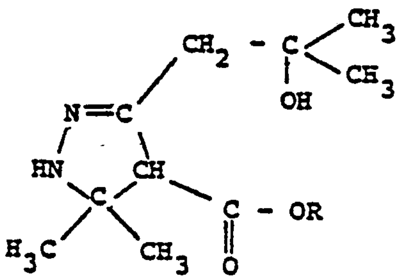

(V)

traite le composé de formule V par un agent d'oxydation en phase hétérogène, pour obtenir le composé de formule VI

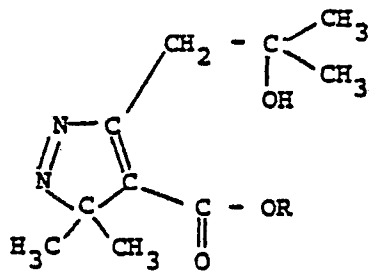

(VI)

et soumet le composé de formule VI à une irradiation pour obtenir le composé de formule I attendu.

Dans un mode d'exécution préféré du procédé de l'invention :

a) la base forte que l'on fait agir avec le composé de formule II est l'hydrure de sodium ou l'amidure de sodium ;

b) l'organolithien est le méthyl ou le butyl lithium ;

c) l'agent d'isomérisation du composé de formule III est l'alumine basique, mais on peut égalament utiliser une autre base telle qu'un hydroxyde alcalin ou un carbonate alcalin, ou encore un agent acide ;

d) l'agent d'oxydation utilisé en phase hétérogène peut être notamment le dioxyde de manganèse ou le peroxyde de nickel ;

e) l'irradiation du composé de formule VI est effectuée à l'aide d'une lampe à vapeur de mercure.

L'invention a également pour objet l'application des composés de formule I à la préparation de précurseurs de l'acide dl cis chrysanthémique, à savoir les composés de formule VII

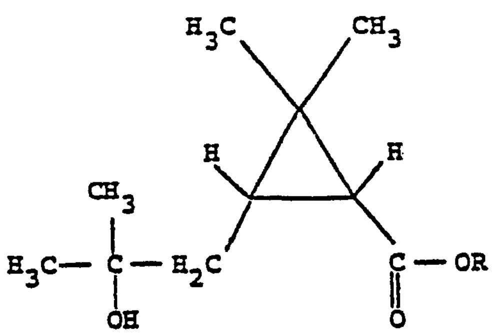

(VII)

de structure cis, dans laquelle R est défini comme précédemment, caractérisée en ce que l'on fait réagir un composé de formule I avec un agent réducteur choisi dans le groupe constitué par l'hydrogène en présence de borure de nickel, de palladium ou de platine et de diimide.

Les composés de formule VII sont connus et décrits notamment dans la demande de brevet japonais publiée sous le numéro 2095/68.

La synthèse de l'acide dl chrysanthémique à partir des composés de formule VII est également connue et peut être effectuée notamment par hydrolyse de l'ester puis déshydratation de la chaine latérale de l'acide obtenu.

On sait l'intérêt que peut présenter l'acide dl cis chrysanthémique, notamment pour préparer des acides cyclopropane carboxyliques à chaîne dihalovinylique dont les esters possèdent des propriétés insecticides remarquables (voir par exemple, les brevets français 2 185 612 et 2 240 914).

La présente invention permet d'accéder à la structure cis chrysanthémique avec un nombre de stades restreint et d'excellents rendements.

Les exemples suivants illustrent l'invention sans toutefois la limiter.

Exemple 1

3,3-diméthyl-2-(2-hydroxy-2-méthylpropyl)-1-cyclopropène-1-carboxylate d'éthyle.

Stade A : 5-hydroxy 5-méthyl-2-(1-méthyléthényl)-3-oxo hexanoate d'éthyle.

On met en suspension 360 mg d'hydrure de sodium à 50 % dans l'huile minérale, dans 50 $cm^3$ de tétrahydrofuranne anhydre. On ajoute goutte à goutte à température ambiante, 3,00 g de 2-(1-méthyléthylidène)-3-oxo butanoate d'éthyle/décrit dans Helv. Chim. Act 54 1797 (1971)/. On porte au reflux pendant une heure, laisse revenir à température ambiante, ajoute à 0 °C 13 $cm^3$ d'une solution 1,6 M de butyllithium dans l'hexane, maintient sous agitation à 0 °C pendant une demi-heure, refroidit à -10 °C et ajoute 1,60 g d'acétone. On verse dans l'eau, extrait à l'éther, lave la phase éthérée avec une solution aqueuse saturée de chlorure de sodium et sèche sur sulfate de magnésium. On concentre à environ 100 $cm^3$ et obtient une solution renfermant le produit attendu en mélange avec son isomère le 5-hydroxy-5-méthyl-2-(1-méthyléthylidène) 3-oxo hexanoate d'éthyle.

Stade B : 5-hydroxy-5-méthyl-2-(1-méthyléthylidène)-3-oxo hexanoate d'éthyle.

On ajoute 15 g d'alumine basique à la solution obtenue ci-dessus, maintient sous agitation pendant une nuit, filtre et évapore le solvant. On chromatographie le résidu sur colonne de silice préparée dans le mélange éther-éther de pétrole (40-60). On élue d'abord au mélange éther-éther de pétrole (50-50) pour éliminer le produit de départ restant, puis au mélange éther-éther de pétrole (80-20). On obtient 1,85 g de produit attendu.

Stade C : 4,5-dihydro-5,5-diméthyl-3(2-hydroxy-2-méthyl propyl)-/1H/-pyrazole-4-carboxylate d'éthyle.

On ajoute 3 $cm^3$ d'acide acétique et 121 mg d'hydrate d'hydrazine à 500 mg du produit obtenu ci-dessus. La température monte spontanément à 40 °C. On distille l'acide acétique en chauffant le mélange jusqu'à atteindre 180 °C lorsque la majeure partie de l'acide acétique a distillé. On refroidit, ajoute de l'eau et extrait au chloroforme. On sèche la phase organique sur sulfate de magnésium, évapore le solvant et obtient 520 mg de produit attendu se présentant sous forme d'un liquide.

Stade D : 3,3-diméthyl-5-(2-hydroxy-2-méthylpropyl)-/3H/-pyrazole-4-carboxylate d'éthyle.

On prépare une suspension de 1,5 g de dioxyde de manganèse dans 50 $cm^3$ de chlorure de méthylène et maintient sous agitation pendant 15 minutes sous gaz inerte. On ajoute 200 mg du produit obtenu ci-dessus et maintient sous agitation pendant 1 heure. On filtre, évapore le solvant et obtient 185 mg de produit attendu.

Stade E : 3,3-diméthyl-2-(2-hydroxy-2-méthylpropyl)-1-cyclopropène-1-carboxylate d'éthyle.

On dissout 350 mg de produit obtenu selon le procédé exposé au stade précédent dans 100 $cm^3$ d'acétate d'éthyle. On y fait barboter un gaz inerte pendant un quart d'heure puis on irradie la solution à l'aide d'une lampe à vapeur de mercure. Après 10 minutes, on obtient un dégagement de 33 $cm^3$ d'azote. On évapore le solvant et obtient 290 mg de produit attendu.

Exemple 2

Cis 2,2-diméthyl-3-(2-hydroxy-2-méthyl propyl)cyclopropane carboxylate d'éthyle.

Dans un appareil à hydrogèner contenant 90 mg de palladium sur charbon à 5 %, on introduit le produit obtenu à l'exemple 1, en solution dans l'acétate d'éthyle. On met sous légère pression d'hydrogène pendant 30 minutes, filtre, évapore le solvant, purifie le produit obtenu par un passage sur colonne de silice préparée dans le mélange éther-hexane (30-70) et obtient 275 mg de produit attendu.

**Revendications**

1. Les composés de formule I

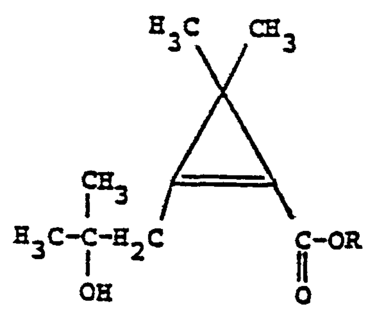

(I)

dans laquelle R représente un radical alcoyle renfermant de 1 à 6 atomes de carbone.

2. Le 3,3-diméthyl-2-(2-hydroxy-2-méthylpropyl)-1-cyclopropène-1-carboxylate d'éthyle.

3. Procédé de préparation des composés de formule I, telle que définie à la revendication 1, caractérisé en ce que l'on fait réagir un composé de formule II

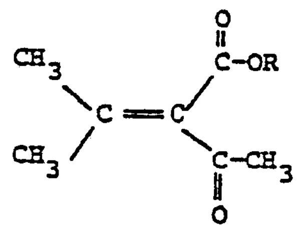

(II)

dans laquelle R est défini comme à la revendication 1, avec une base forte puis avec un organolithien, fait réagir le composé obtenu avec de l'acétone pour obtenir le composé de formule III

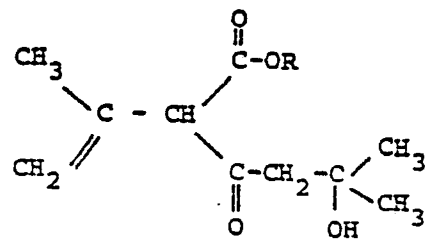

(III)

isomérisé le composé de formule III par un agent basique ou par un agent acide pour obtenir le composé de formule IV

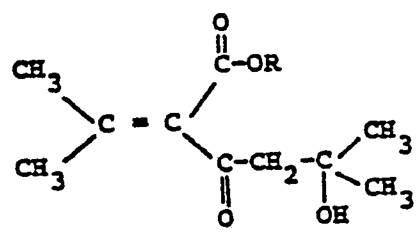

(IV)

fait réagir le composé de formule IV avec de l'hydrazine dans l'acide acétique, pour obtenir le composé de formule V

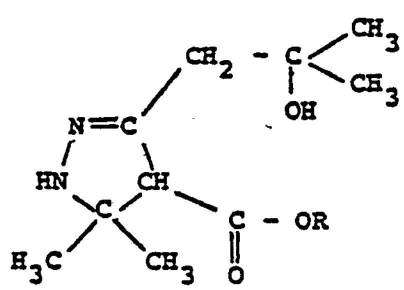

(V)

traite le composé de formule V par un agent d'oxydation en phase hétérogène, pour obtenir le composé de formule VI

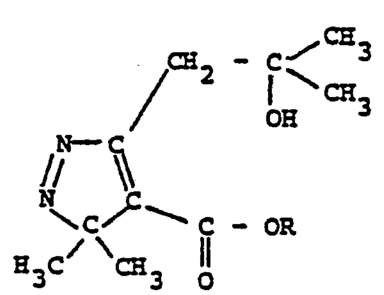

(VI)

et soumet le composé de formule VI à une irradiation pour obtenir le composé de formule I attendu.

4. Application des composés de formule I, telle que définie à la revendication 1, à la préparation des composés de formule VII

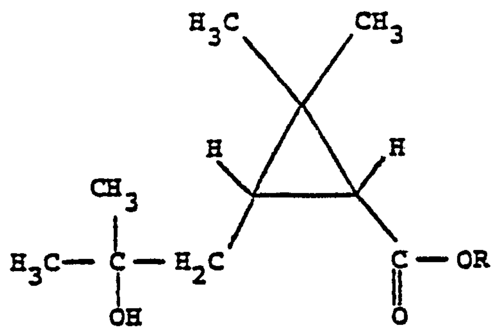

(VII)

de structure cis, dans laquelle R est défini comme à la revendication 1, caractérisé en ce que l'on fait réagir un composé de formule I avec un agent réducteur choisi dans le groupe constitué par l'hydrogène en présence de borure de nickel, de palladium ou de platine et le diimide.

**Claims**

1. The compounds of formula I

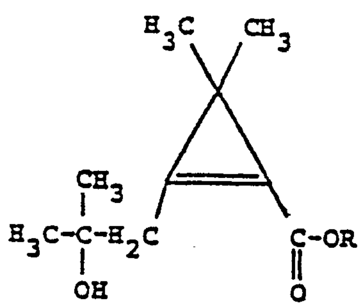

(I)

in which R represents an alkyl radical containing from 1 to 6 carbon atoms.

2. Ethyl 3,3-dimethyl-2-(2-hydroxy-2-methylpropyl)-1-cyclopropene-1-carboxylate.

3. Process for preparing the compounds of formula I, as defined in Claim 1, characterized in that a compound of formula II

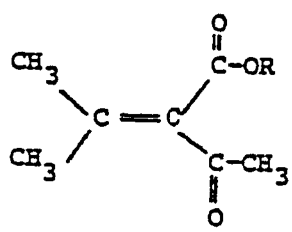

(II)

in which R is defined as in Claim 1, is reacted with a strong base then with an organolithium derivative, the compound obtained is reacted with acetone to obtain the compound of formula III

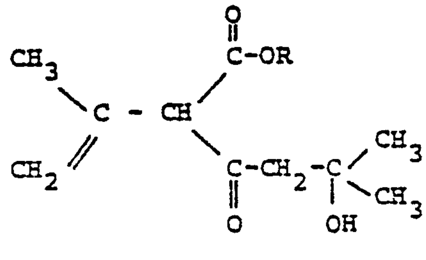

(III)

the compound of formula III is isomerized with a basic agent or with an acidic agent to obtain the compound of formula IV

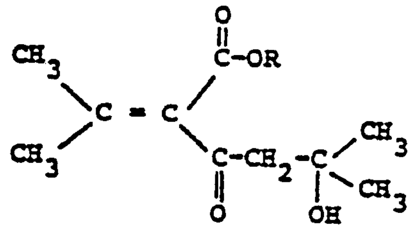

(IV)

the compound of formule IV is reacted with hydrazine in acetic acid, to obtain the compound of formula V

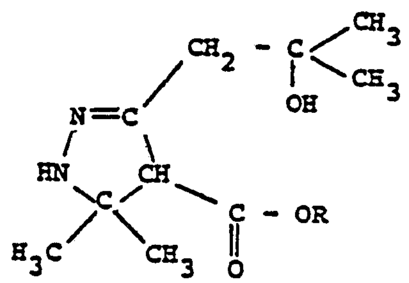

(V)

the compound of formula V is treated with an oxidizing agent in heterogeneous phase, to obtain the compound of formula VI

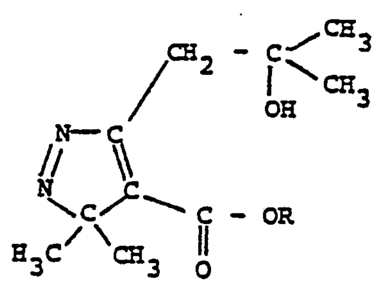

(VI)

and the compound of formula VI is subjected to irradiation to obtain the expected compound of formula I.

4. Use of the compounds of formula I, as defined in claim 1, in the preparation of the compounds of formula VII

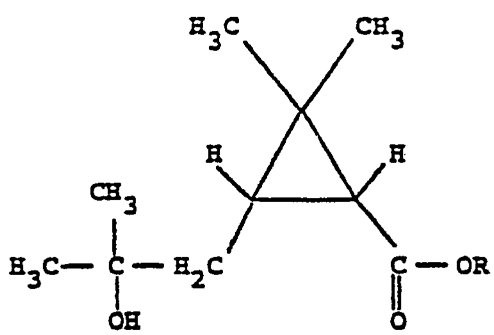

(VII)

of cis structure, in which R is defined as in Claim 1, characterized in that a compound of formula I is reacted with a reducing agent selected from the group constituted by hydrogen in the presence of the boride of nickel, of palladium or of platinum, and diimide.

**Ansprüche**

1. Verbindungen der Formel I

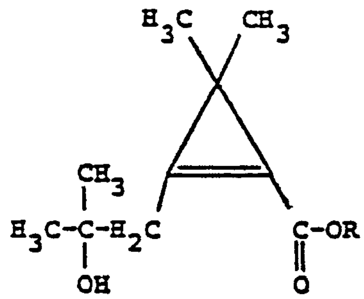

(I)

worin R einen Alkylrest mit 1 bis 6 Hohlenstoffatomen bedeutet.

2. 3,3-Dimethyl-2-(2-hydroxy-2-methylpropyl)-1-cyclopropen-1-carbonsäureäthylester.

3. Verfahren zur Herstellung der Verbindungen der Formel I gemäß Anspruch 1, dadurch gekennzeichnet, daß man eine Verbindung der Formel II

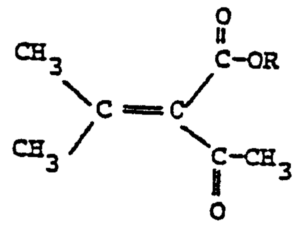

(II)

worin R wie in Anspruch 1 definiert ist, mit einer starken Base und anschließend mit einer Organolithiumverbindung umsetzt, die erhaltene Verbindung mit Aceton umsetzt, um die Verbindung der Formel III

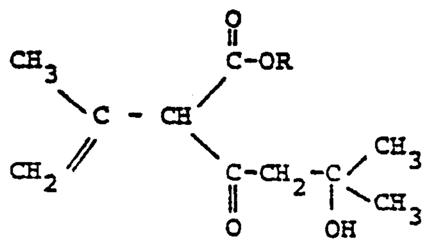

(III)

zu erhalten, die Verbindung der Formel III mit einem basischen Mittel oder mit einem sauren Mittel isomerisiert, um die Verbindung der Formel IV

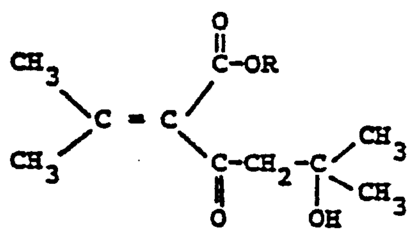

(IV)

zu erhalten, die Verbindung der Formel IV mit Hydrazin in Essigsäure umsetzt, um die Verbindung der Formel V

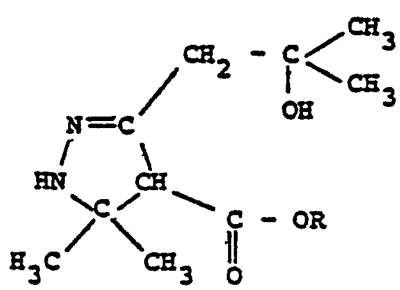

(V),

zu erhalten, die Verbindung der Formel V mit einem Oxidationsmittel in heterogener Phase behandelt, um die Verbindung der Formel VI

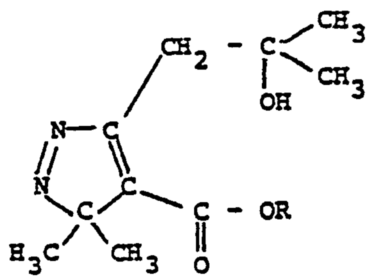

(VI)

zu erhalten, und die Verbindung der Formel VI einer Bestrahlung unterzieht, um die erwartete Verbindung der Formel I zu erhalten.

4. Verwendung der Verbindungen der Formel I gemäß Anspruch 1 zur Herstellung von Verbindungen der Formel VII

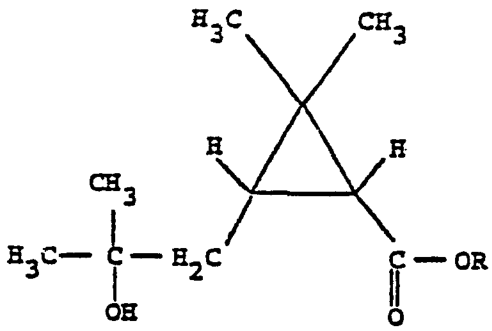

(VII)

mit cis-Struktur, worin R wie in Anspruch 1 definiert ist, dadurch gekennzeichnet, daß man eine Verbindung der Formel I mit einem Reduktionsmittel, ausgewählt aus der Gruppe bestehend aus Wasserstoff in Gegenwart von Nickel-, Palladium- oder Platinborid und Diimid, umsetzt.